# FASCICULE DE BREVET EUROPEEN

(11) **EP 1 456 502 B1**
(45) Date de publication et mention de la délivrance du brevet: **27.08.2008**
(21) Numéro de dépôt: 02799108.2
(22) Date de dépôt: 20.12.2002
(51) Int. Cl.: A61Q 19/00, B01F 17/00, C09K 8/588, C11D 3/37, A61Q 5/00

(54) **UTILISATION DE POLYMERES STATISTIQUES AMPHIPHILES CHARGES POUR EPAISSIR DES PHASES COMPRENANT DES MICELLES GEANTES DE TENSIOACTIFS ET COMPOSITION AQUEUSE LES COMPRENANT**
VERWENDUNG VON GEFÜLLTEN AMPHIPHILEN STATISTISCHEN POLYMEREN ZUM EINDICKEN VON RIESENMIZELLEN ENTHALTENDEN PHASEN UND WÄSSRIGE ZUSAMMENSETZUNG DAVON
USE OF CHARGED AMPHIPHILIC STATISTIC POLYMERS FOR THICKENING PHASES COMPRISING GIANT MICELLES OF SURFACTANTS AND AQUEOUS COMPOSITIONS COMPRISING SAME

(30) Priorité: 21.12.2001 FR 0116712
(43) Date de publication de la demande: 15.09.2004
(73) Titulaire: RHODIA CHIMIE, 92512 Boulogne Billancourt Cedex (FR)
(72) Inventeur: BAVOUZET, Bruno, F-75010 Paris (FR); CHAPON, Pascal, F-31400 Toulouse (FR)
(74) Mandataire: Boittiaux, Vincent
(86) Numéro de dépôt international: PCT/FR2002/004498
(87) Numéro de publication internationale: WO 2003/054350

(56) Documents cités:
- EP-A- 1 068 857
- FR-A- 2 807 649
- US-A- 4 728 696

## Description

La présente invention a pour objet l'utilisation de polymères statistiques chargés comme agent épaississant de compositions aqueuses comprenant un ou plusieurs tensioactifs organisés en phase de micelles géantes, ainsi que les compositions comprenant de telles phases organisées épaissies par lesdits polymères.

Le domaine de la présente invention est tourné vers les formulations aqueuses tensioactives formant des micelles géantes.

Dans de nombreux domaines mettant en oeuvre des formulations tensioactives, une attention toute particulière est portée sur la viscosité de la formulation. En effet, on souhaite pouvoir disposer de formulations présentant des viscosités relativement élevées que ce soit pour des raisons d'ordre purement technique, comme par exemple te maintien de particules en suspension, ou bien encore pour des raisons tournées vers l'utilisateur, telles que par exemple, la facilité, le confort de distribution et de mise en oeuvre de la formulation, un toucher sensoriel particulier, voire pour l'ensemble de ces raisons.

La difficulté réside dans le fait que les formulations tensioactives aqueuses présentent des viscosités jugées trop faibles pour bon nombre d'applications.

Diverses solutions ont été apportées à cet inconvénient.

Ainsi, on a utilisé en tant qu'agent modificateur de rhéologie, des sels, comme typiquement le chlorure de sodium. Cependant, la quantité de ce type de composé ne peut être trop élevée au risque d'avoir des effets néfastes sur la formulation.

L'utilisation de polymères tels que les dérivés de polysaccharides modifiés ou non, tels que les polymères synthétiques du type des polyacrylates réticulés par exemple, a aussi été développée. Les difficultés rencontrées lors de l'emploi de ces additifs, sont dues notamment au fait qu'ils doivent être employés, pour certains, en quantités importantes, qu'ils peuvent être relativement complexes de mise en oeuvre, et/ou encore que leur domaine d'utilisation est limité car leur stabilité dépend du pH et de la concentration en sel de la formulation.

Le document US 4728696 décrit des polymères associatif, comprenant un monomère amphiphile, capable d'augmenter la viscosité en présence de sel.

Les micelles géantes représentent enfin une catégorie de phase organisée de tensioactifs recherchée, car entre autres avantages, elles confèrent à la formulation des propriétés rhéologiques particulières. Notamment, elles développent des viscosités plus élevées que des solutions de tensioactifs qui en seraient dépourvues, lorsque l'on souhaite limiter les concentrations en tensioactifs et/ou en sel..

Le problème est que l'augmentation de viscosité apportée grâce à la présence des micelles géantes peut ne pas être considérée comme suffisante.

On recherche donc toujours des additifs épaississants à ajouter à des compositions aqueuses tensioactives, additifs qui notamment seraient stables dans diverses conditions de pH et de concentration en sels et qui seraient efficaces à des concentrations relativement faibles.

On recherche de plus des additifs qui permettraient d'obtenir des formulations présentant des caractéristiques additionnelles spécifiques. Ainsi, on souhaite pouvoir disposer d'additifs qui ne rendent pas impossible l'obtention de formulations transparentes, ou encore qui apportent des propriétés fonctionnelles supplémentaires à la formulation comme un effet conditionneur par exemple, caractéristiques recherchées plus spécialement dans le domaine des formulations destinées au traitement de la peau et/ou du cheveu. Par ailleurs, dans d'autres domaines, tels que notamment celui de l'exploitation de gisements pétroliers ou de gaz, on souhaite pouvoir mettre en oeuvre des additifs qui apportent à la formulation dans laquelle ils entrent, une résistance thermique améliorée.

Ces buts et d'autres sont atteints par la présente invention qui a donc pour premier objet l'utilisation comme agent épaississant d'une composition aqueuse comprenant au moins 5 % en poids d'un ou plusieurs tensioactif(s) organisé(s) en phase comprenant des micelles géantes dont la longueur moyenne représente au moins 4 fois le diamètre moyen, d'au moins un polymère amphiphile statistique ioniquement chargé soluble dans la composition aqueuse ; ledit polymère amphiphile étant utilisé avec une teneur telle que la viscosité de la composition aqueuse comprenant le polymère amphiphile est au moins trois fois supérieure à celle de la composition dépourvue de polymère amphiphile et à celle d'une solution aqueuse comprenant le polymère amphiphile seul.

Elle a de même pour objet une composition aqueuse comprenant au moins 5 % en poids d'un ou plusieurs tensioactif(s) organisé(s) en phase comprenant des micelles géantes dont la longueur moyenne représente au moins 4 fois le diamètre moyen, et comprenant au moins un polymère amphiphile statistique ioniquement chargé et soluble dans la composition aqueuse ; ledit polymère amphiphile étant utilisé avec une teneur telle que la viscosité de la composition aqueuse comprenant le polymère amphiphile est au moins trois fois supérieure à celle de la composition dépourvue de polymère amphiphile et à elle d'une solution aqueuse comprenant le polymère amphiphile seul.

On a en effet constaté de manière totalement inattendue que des formulations aqueuses comprenant une phase organisée de tensioactif(s), du type des micelles géantes, pouvaient être épaissies de manière efficace lorsqu'on leur ajoutait un polymère amphiphile statistique chargé. Et ceci est d'autant plus surprenant que ledit polymère n'est pas un agent épaississant de solutions aqueuses en lui-même. Ainsi, contrairement aux systèmes classiques comprenant des polymères ayant des propriétés intrinsèques d'agent épaississant, les polymères mis en oeuvre dans le cadre de la présente invention ne sont pas des épaississants de phase aqueuse en tant que tels, mais augmentent pourtant de façon significative les propriétés rhéologiques (viscosité, visco-élasticité) apportées initialement par les micelles géantes.

On a de plus observé que ledit polymère statistique, outre son rôle d'agent épaississant de formulation comprenant des micelles géantes, avait dans les mêmes conditions, un rôle d'agent dispersant de particules.

Selon une autre caractéristique avantageuse de l'invention, on a aussi remarqué que ledit polymère, toujours dans les mêmes conditions, avait un rôle supplémentaire d'agent émulsifiant de phase non miscible à la formulation aqueuse et/ou d'agent stabilisant d'une telle phase.

Enfin, on a constaté que ledit polymère statistique, dans les mêmes conditions, avait un rôle supplémentaire d'aide au dépôt de particules ou d'émulsions sur une surface.

Mais d'autres avantages et caractéristiques de l'invention apparaîtront plus clairement à la lecture de la description et des exemples qui vont suivre.

L'invention concerne donc l'épaississement de compositions aqueuses comprenant au moins un tensioactif avec une teneur d'au moins 5 % en poids par rapport au poids de la composition, de préférence une teneur comprise entre 5 et 15 % en poids.

Il est précisé que les compositions sont plus particulièrement des formulations utilisables dans divers domaines et notamment dans le domaine du traitement de la peau et/ou du cheveu, ou encore dans le domaine de l'exploitation des gisements pétroliers ou de gaz.

Ces compositions présentent une phase organisée de tensioactif(s) qui est du type "micelles géantes".

Ce type de phase organisée développe intrinsèquement une viscosité particulière mais qui reste toujours insuffisante.

Au sens de l'invention, le terme "micelles géantes" désigne des objets dispersés dans la composition aqueuse et dont la longueur moyenne représente au moins quatre fois le diamètre moyen de ces vésicules. Ces objets sont bien connus de l'homme du métier, et l'on y fait référence dans les publications entre autres sous les noms de "micelles sous forme de vermicelles" (ou worm-like micelles) ou de "polymères vivants" (ou living polymers).

Ces micelles peuvent notamment être observées, par exemple, au moyen d'un microscope électronique.

Plus particulièrement, la longueur moyenne des micelles géantes est habituellement supérieure ou égale à 10 fois celle du diamètre. De préférence, elle est supérieure ou égale à 100 fois celle du diamètre.

Le ou les tensioactifs sont choisis parmi ceux qui donnent des phases organisées de type micelles géantes, lorsqu'ils sont en solution aqueuse.

Selon un mode de réalisation particulier de l'invention, la composition aqueuse comprend au moins un tensioactif qui à une concentration dans l'eau supérieure ou égale à 5 % en poids donne une phase organisée de type micelles géantes, dans les conditions d'utilisation de la composition (c'est-à-dire dans les conditions de concentrations, de pH, de force ionique, de température).

Le choix du tensioactif ne représente pas en soi une difficulté particulière pour l'homme du métier.

Il y a néanmoins plusieurs cas de figure.

Selon une première variante, le tensioactif (ou tensioactif principal) est choisi parmi les espèces, qui, seules, sont capables de former une phase micelles géantes dans les conditions de concentration, température, pH et force ionique de la composition.

Pour définir le tensioactif convenable, l'homme de l'art peut procéder en traçant le diagramme de phase pour déterminer dans quel domaine de concentration la phase souhaitée existe, si tel est le cas, et ensuite constater si ce domaine est compatible avec les conditions de température, pH, force ionique, température de la composition.

Selon cette première variante, il peut être avantageux d'associer au tensioactif principal choisi, soit au moins un sel, soit au moins un co-tensioactif, voire la combinaison de ces deux types de composés. L'intérêt de l'emploi de ce type d'additifs est qu'ils représentent un moyen de diminuer la teneur en tensioactif principal pour obtenir la phase micelles géantes, ou bien encore dit autrement, d'augmenter le niveau de viscosité de la composition à concentration égale en tensioactif principal.

En ce qui concerne le choix du co-tensioactif possible, il est de préférence choisi en fonction de la nature du tensioactif principal précité, et peut notamment être un tensioactif de charge nette opposée à celle du tensioactif principal, ou bien encore de type amphotère. Il peut aussi être choisi parmi les tensioactifs non ioniques.

Selon une deuxième variante de l'invention, le tensioactif principal est choisi parmi ceux qui permettent d'accéder à une phase de micelles géantes en présence d'au moins un sel et/ou d'au moins un co-tensioactif.

L'homme de l'art, selon cette deuxième variante, procèdera de manière similaire à la précédente, pour déterminer la combinaison tensioactif / sel et/ou co-tensioactif qui permettra, dans les conditions de concentration, température, pH et force ionique de la composition, d'accéder à la phase micelles géantes.

A titre d'exemples de tensioactifs convenables utilisables en tant que tensioactif principal, on peut notamment citer les tensioactifs non ioniques dérivant d'alcools aliphatiques, linéaire ou ramifiés, comprenant 6 à 24 atomes de carbone et comprenant des motifs oxyéthylène et éventuellement oxypropylène dans une teneur telle que le tensioactif présente une valeur de HLB d'au moins 10. A titre purement illustratif, le nombre de motifs oxyéthylène et éventuellement oxypropylène est compris entre 2 et 10.

Des organisations de tensioactifs en phase comprenant des micelles géantes particulièrement intéressantes sont des organisations telles la composition aqueuse présente des propriétés viscoélastiques. De telles compositions et/ou organisations sont notamment décrites dans la demande de brevet internationale publiées sous le numéro WO98/56497. L'addition du polymère amphiphile chargé à ces compostions augmente la viscosité, la composition conservant par ailleurs des propriétés viscoélastiques.

Des tensioactifs avantageux pour l'obtention d'une organisation en phase comprenant des micelles géantes sont les tensioactifs amphotères or zwitterioniques. Des exemples de tensioactifs zwitterioniques pouvant être utilisés comprennent les composés de formule R¹R²R³N⁺-R⁴COO⁻, où R¹ est un groupe hydrophobe alkyle, alkylarylalkyle, alkoxyalkyle, alkylaminoalkyl, allylamidialkyle, où la partie alkyle est de C₁₂ à C₂₄ , R² et R³, identiques ou différents sont des chaînes aliphatiques (c'est à dire dont l'atome lié à l'atome d'azote ne fait pas partie d'un groupe aromatique), de C₁ à C₃₀, par exemple un groupe methyle, ethyle, benzyl, hydroxyethyle, hydroxyethyle, acetate ou propionate, et R⁴ est un groupe hydrocarboné divalent tel qu'un groupe méthylène ou éthylène. Des exemples de tensioactifs amphotères pouvant être utilisés comprennent les composés de formule R¹R²NH⁺-R⁴COO⁻, où R¹, R² et R⁴ sont tels que mentionnés précédemment.

Il peut être aussi possible de mettre en oeuvre, comme tensioactif principal, au moins un tensioactif choisi parmi les espèces anioniques suivantes :
- les alkylesters sulfonates, par exemple de formule R-CH(SO₃M)-COOR', où R représente un radical alkyle en C₈-C₂₀, de préférence en C₁₀-C₁₆, R' un radical alkyle en C₁-C₆, de préférence en C₁-C₃ et M un cation alcalin (sodium, potassium, lithium), ammonium substitué ou non substitué (méthyl-, diméthyl-, triméthyl-, tétraméthylammonium, diméthylpipéridinium ...) ou dérivé d'une alcanolamine (monoéthanolamine, diéthanolamine, triéthanolamine ...). On peut citer tout particulièrement les méthyl ester sulfonates dont le radical R est en C₁₄-C₁₆ ; les alkylbenzènesulfonates, plus particulièrement en C₉-C₂₀, les alkylsulfonates primaires ou secondaires, notamment en C₈-C₂₂, les alkylglycérol sulfonates, les acides polycarboxyliques sulfonés, comme par exemple ceux décrits dans GB 1082179, les sulfonates de paraffine ;
- les alkylsulfates par exemple de formule ROSO₃M, où R représente un radical alkyle ou hydroxyalkyle en C₁₀-C₂₄, de préférence en C₁₂-C₂₀ ; M représentant un atome d'hydrogène ou un cation de même définition que ci-dessus, ainsi que leurs dérivés polyalcoxylés (éthoxylés (OE), propoxylés (OP), ou leurs combinaisons), comme par exemple le dodécylsulfate de sodium ;
- les alkyléthersulfates par exemple de formule RO(CH₂CH₂O)ₙSO₃M où R représente un radical alkyle ou hydroxyalkyle en C₁₀-C₂₄, de préférence en C₁₂-C₂₀ ; M représentant un atome d'hydrogène ou un cation de même définition que ci-dessus, n variant généralement de 1 à 4, ainsi que leurs dérivés polyalcoxylés (éthoxylés (OE), propoxylés (OP), ou leurs combinaisons), comme par exemple le laurylethersulfate avec n = 2.
- les alkylamides sulfates, par exemple de formule RCONHR'OSO₃M où R représente un radical alkyle en C₂-C₂₂, de préférence en C₆-C₂₀, R' un radical alkyle en C₂-C₃, M représentant un atome d'hydrogène ou un cation de même définition que ci-dessus, ainsi que leurs dérivés polyalcoxylés (éthoxylés (OE), propoxylés (OP), ou leurs combinaisons) ;
- les sels d'acides gras saturés ou insaturés, par exemple comme ceux en C₈-C₂₄, de préférence en C₁₄-C₂₀, les N-acyl N-alkyltaurates, les alkyliséthionates, les alkylsuccinamates et alkylsulfosuccinates, les monoesters ou diesters de sulfosuccinates, les N-acyl sarcosinates, les polyéthoxycarboxylates ; et
- les phosphates esters d'alkyle et/ou d'alkyléther et/ou d'alkylaryléther.

Pour ce qui concerne les sels susceptibles d'être utilisés, on peut citer les sels d'acides inorganiques, comme les sels d'acide chlorhydrique (sodium, potassium). Parmi les sels convenables, figurent aussi les sels d'acides organiques dont le contre-ion est choisi parmi ceux capables de diminuer la valeur de la HLB (balance hydrophile /lipophile) du tensioactif principal. Comme exemple particulier de ce type de sels, on peut citer le chlorure de salicylate de cétyltriméthylammonium.

Si un sel est présent, et quelle que soit la variante choisie, sa teneur représente habituellement une concentration comprise entre 0,1 et 5 %en poids de la composition.

En ce qui concerne le co-tensioactif possible, celui-ci peut être choisi parmi les tensioactifs suivants, utilisés seuls ou combinés :
- les alkylamphoacétates et alkylamphodiacétates répondant plus généralement à la formule : RCO-NHa(CH₂COONa)₁₋ₐ-CH₂CH₂-N(CH₂COONa)-CH₂CH₂-OH ; formule dans laquelle R représente un radical alkyle ou alcényle de 10 à 24 atomes de carbone, a est égal à 0 ou 1 ; R représente plus particulièrement des chaînes coco et lauryle (par exemple les composés Miranol C2M et Miranol Ultra C32 de la société Rhodia Chimie) ;
- les alkylampho-propionates ou -dipropionates, plus particulièrement de formule : RCO-NHₐ(CH₂CH₂COONa)₁₋ₐ-CH₂CH₂-N(CH₂CH₂COONa)-CH₂CH₂-OH ; les produits Miranol C2M SF de la société Rhodia Chimie en sont des exemples ;
- les alkyl amphohydroxypropyl-sultaines ; comme par exemple les composés Miranol CS de la société Rhodia Chimie.

Parmi les tensioactifs zwittérioniques utilisables en tant que co-tensioactif, on peut notamment citer, seuls ou en mélanges :
- les bétaïnes, par exemple de formule R(R')₃N⁺-COO⁻; formule dans laquelle le radical R représente un radical alkyle ou alcényle comprenant 10 à 24 atomes de carbone, R', identiques ou non, représentent un radical alkyle ou alcényle ayant de 1 à 4 atomes de carbone. A titre d'exemple, on peut citer particulièrement la lauryl bétaine (Mirataine BB de la société Rhodia Chimie) ;
- les sulfo-bétaïnes, par exemple de formule suivante : R(R')₃N⁺-SO₃- ; formule dans laquelle les radicaux R et R' ont la même signification que ci-dessus ;
- les amidoalkylbétaïnes, notamment de formule suivante : RCO-NHR'-N⁺(R')₃-COO⁻; formule dans laquelle les radicaux R et R' ont la même signification que ci-dessus. La cocamidopropyl bétaine (Mirataine BDJ de la société Rhodia Chimie) est un exemple de cetype de composé ;
- les sulfo-amidoalkylbétaïnes, par exemple de formule suivante : RCO - NHR'-N⁺ (R')₃-SO₃⁻ ; R et R' ayant la même signification qu'auparavant.

Au cas où un tensioactif non ionique est employé en tant que co-tensioactif, on peut citer entre autres
- les alcools gras éventuellement alcoxylés
- les mono-, di- et tri-glycérides alcoxylés
- les acides gras alcoxylés
- les esters de sorbitan alcoxylés
- les amines grasses alcoxylées
- les alkylphénols alcoxylés
- les alkylpolyglucosides,
seuls ou en mélange.

Les alcools gras éventuellement alcoxylés comprennent généralement de 4 à 24 atomes de carbone, les motifs alcoxylés étant exclus de ces nombres.

Les mono-, di- et tri-glycérides alcoxylés peuvent être des mono-, di- et tri-glycérides d'origine végétale ou animale.

Les esters de sorbitan éventuellement alcoxylés sont des esters du sorbitol cyclisés d'acide gras comprenant de 10 à 20 atomes de carbone comme l'acide laurique, l'acide stéarique ou l'acide oléïque.

Les amines grasses alcoxylées ont généralement de 10 à 22 atomes de carbone, les motifs alcoxylés étant exclus de ces nombres.

Les alkylphénols alcoxylés ont généralement un ou deux groupes alkyles, linéaires ou ramifiés, ayant 4 à 12 atomes de carbone. A titre d'exemple on peut citer notamment les groupes octyle, nonyle ou dodécyle.

Les alkylpolyglucosides sont plus précisément des tensioactifs, oligomériques, non ioniques, qui se trouvent sous la forme d'acétals d'alcools gras (de préférence en C₈-C₁₆) et de sucres (glucose). Ils sont notamment obtenus par réaction chimique à partir d'amidon, de graisses. De tels tensioactifs sont par exemple commercialisés par la société Henkel sous les dénominations Plantaren^{®}.

Au cas où un co-tensioactif est présent, quelle que soit la variante retenue, sa teneur est telle que le rapport pondéral co-tensioactif / tensioactif principal est inférieur à 50/50, de préférence compris entre 0,1/99,9 et 40/60.

Le polymère statistique entrant dans la composition aqueuse est tout d'abord choisi parmi ceux qui sont chargés et solubles dans la composition aqueuse. On précise qu'il ne s'agit de préférence pas d'un polymère réticulé ou branché. Il s'agit de préférence d'un polymère linéaire.

Ledit polymère amphiphile mis en oeuvre dans le cadre de la présente invention peut être soit un polymère de type polyanion, c'est-à-dire un polymère qui, pour ce qui concerne les monomères chargés, ne porte que des charges ioniques négatives ; soit un polymère de type polycation, c'est-à-dire ne portant que des charges ioniques positives ; ou encore un polymère de type polyampholyte, c'est-à-dire portant à la fois des charges ioniques positives et négatives.

Il est à noter que l'existence de charge(s) ionique(s) est observée dans les conditions de température, de pH et de force ionique de la composition aqueuse.

Par ailleurs la solubilité du polymère amphiphile dépend du pH, des concentrations respectives des divers éléments constitutifs, ainsi que de la température d'utilisation et de préparation de ladite composition aqueuse.

De plus, un polymère sera dit soluble au sens de l'invention, si la composition aqueuse comprenant ledit polymère, au bout de 2 heures ne présenté pas de séparation macroscopique de phases, à environ 20°C.

Par ailleurs, le polymère utilisable dans l'invention peut se trouver en fait sous la forme d'un oligomère, c'est-à-dire d'une espèce dont le degré de polymérisation moyen est compris entre 3 et 10, ou d'un polymère, c'est-à-dire d'une espèce dont le degré de polymérisation moyen est supérieur à 10. Dans la suite, on utilisera seulement le terme de polymère, sachant qu'il recouvre les deux possibilités qui viennent d'être énoncées.

Ainsi, le polymère, dans les conditions de pH de la composition aqueuse, peut présenter un coefficient f d'au moins 0,05, plus particulièrement compris entre 0,05 et 2. Il est rappelé que ce coefficient f représente la fraction de charge totale du polymère amphiphile, et qu'il correspond à la somme du nombre de moles de charges théoriques de monomères portant une charge (anionique, cationique) dans le polymère, divisée par la somme du nombre de moles total théorique de monomères dans ledit polymère amphiphile.

De plus, selon un mode de réalisation particulier de l'invention, le polymère amphiphile présente un coefficient Δf comprise entre -0,5 et 0,5.

Il est rappelé que le coefficient Δf correspond à la différence entre le nombre de moles théoriques de charges cationiques et le nombre de moles théoriques de charges anioniques du polymère, l'ensemble étant divisé par la somme du nombre de moles de monomères théorique total du polymère.

Plus particulièrement, selon un premier mode de réalisation de l'invention, le polymère amphiphile est choisi parmi ceux comprenant une charge nette neutre. En d'autres termes, les polymères entrant dans ce mode de réalisation, présente un coefficient Δf compris entre -0,1 et 0,1 (le nombre de moles théoriques de charges cationiques dans le monomère est voisin de celui du nombre de moles théoriques de charges anioniques).

Selon un second mode de réalisation de l'invention, le polymère amphiphile possède une charge nette opposée à celle du tensioactif principal.

Une première variante de ce second mode consiste à mettre en oeuvre un polymère dont la valeur de Δf est comprise entre 0,1 exclu et 0,5. Selon cette première variante, le polymère possède un nombre de mole théorique de charges cationiques dans le polymère amphiphile, supérieur à celui des charges anioniques. Dans ce cas, ce polymère amphiphile est de préférence mis en oeuvre lorsque le ou les tensioactifs à l'origine de la phase micelles géantes, sont de type anionique.

Par ailleurs, et de manière symétrique, la deuxième variante de ce second mode consiste à mettre en oeuvre un polymère dont la valeur de Δf est comprise entre -0,5 et -0,1 exclu. Selon cette variante, le polymère possède un nombre de moles théorique de charges anioniques dans le polymère amphiphile, supérieur à celui des charges cationiques. Dans ce cas, ce polymère amphiphile est de préférence mis en oeuvre lorsque le ou les tensioactifs à l'origine de la phase micelles géantes, sont de type cationique.

Le polymère amphiphile employé comme agent épaississant comprend par ailleurs des motifs hydrophobes. Ces motifs, comprenant au moins 6 atomes de carbone, de préférence sont plus particulièrement de type alkyle, linéaire ou ramifié, aryle, éventuellement porteur d'un ou plusieurs radicaux alkyles, linéaires ou non. Il est précisé que le radical aryle peut être rattaché directement au squelette du polymère ou bien y être rattaché par l'intermédiaire d'un radical alkyle.

De préférence, les motifs hydrophobes, identiques ou non, sont des radicaux alkyle ramifiés comprenant 6 à 24 atomes de carbone, de préférence 6 à 10 atomes de carbone, des radicaux aryle, de type benzyle éventuellement porteur d'un ou plusieurs radicaux alkyles, linéaires ou non.

Les monomères à partir desquels sont obtenus les motifs hydrophobes peuvent être non ioniques, mais aussi porter une charge ionique (anionique ou cationique).

A titre d'exemples de monomères non ioniques convenables à partir desquels les motifs hydrophobes du polymère statistique peuvent être obtenus, on peut citer seuls ou en mélanges :
- les esters des acides mono- ou poly- carboxyliques, linéaires, ramifiés, cycliques ou aromatiques, comprenant au moins une insaturation éthylénique ;
- les nitriles α(β-éthyléniquement insaturés, les esters vinyliques, les monomères vinylaromatiques,
les monomères hydrocarbonés, linéaires ou ramifiés, aromatiques ou non, comprenant au moins une insaturation éthylénique.

A titre d'exemples préférés de tels monomères, on peut citer les esters d'acide (méth)acrylique avec un alcool comprenant au moins 6 à 24, de préférence 6 à 10 atomes de carbone, comme l'acrylate ou le méthacrylate de 2-éthylhexyl ; le styrène, l'a-méthylstyrène, le vinyltoluène, seuls ou en mélanges.

En ce qui concerne les monomères hydrophobes cationiques, on peut citer entre autres les monomères suivants, comprenant au moins un radical alkyle ou aromatique possédant au moins 6 atomes de carbone et du type :
- les (méth)acrylates d'aminoalkyle, les (méth)acrylamides d'aminoalkyle, ou leurs sels ;
- les monomères comprenant au moins une fonction amine secondaire, tertiaire ou quaternaire, ou un groupe hétérocyclique contenant un atome d'azote, ou leurs sels ;
- les sels d'ammonium de diallyldialkyle ;
seuls ou en mélanges, dans lesquels le groupe alkyle comprend 6 à 24 atomes de carbone, de préférence 6 à 10.

S'ils se présentent sous la forme de sels, ces derniers sont de préférence choisis de telle sorte que le contre-ion soit un halogénure comme par exemple un chlorure, ou un sulfate, un hydrosulfate, un alkylsulfate, un phosphate, un citrate, un formiate, un acétate.

De plus, l'ion ammonium comprend au moins un radical alkyle ou aryle éventuellement porteur d'un ou plusieurs radicaux alkyles, possédant au moins 6 atomes de carbone.

Pour ce qui a trait aux monomères anioniques, ces derniers sont plus particulièrement choisis parmi ceux comprenant au moins un radical alkyle ou aromatique possédant au moins 6 atomes de carbone et portant au moins une fonction carboxylique, sulfonique, sulfurique, phosphonique, phosphorique, sulfosuccinique, leurs sels ou précurseurs.

Plus particulièrement, les motifs anioniques du copolymère sont obtenus à partir d'au moins un monomère choisi parmi les acides carboxyliques linéaires, ramifiés, cycliques ou aromatiques, les acides vinyl carboxyliques linéaires, ramifiés, cycliques ou aromatiques, seuls ou en mélanges, leurs dérivés sulfoniques ou phosphoniques.

De préférence, le(s) motif(s) hydrophobe(s) du polymère statistique sont obtenus à partir de monomère(s) hydrophobe(s) non ionique(s).

De préférence, le nombre de moles théorique de monomères hydrophobes présent dans la composition du polymère amphiphile est d'au moins 5 % par rapport au nombre de moles total théorique de monomères du polymère.

Dans le cas où les motifs hydrophobes sont des radicaux alkyles, le nombre de moles théorique de monomères hydrophobes présent dans la composition du polymère amphiphile est compris entre 10 et 50% en poids par rapport au nombre de moles total théorique de monomères du polymère, de préférence entre 25 et 40 %.

Dans le cas où les motifs hydrophobes sont des radicaux aryles, le nombre de moles théorique de monomères hydrophobes présent dans la composition du polymère amphiphile est compris entre 5 et 15% en poids par rapport au nombre de moles total théorique de monomères du polymère.

Il est précisé que les teneurs molaires réelles dans le polymère, déterminées par RMN par exemple, sont en accord à ± 20% près avec les teneurs molaires théoriques qui viennent d'être mentionnées.

Le polymère amphiphile comprend par ailleurs des motifs hydrophiles, qui peuvent être soit non ioniques, soit anioniques, soit cationiques, soit amphotères.

Les monomères à partir desquels sont obtenus les motifs hydrophiles sont plus particulièrement choisis parmi ceux qui, une fois homopolymérisé avec un degré de polymérisation compris entre 40 et 100, donnent un polymère soluble dans les conditions de température (15-35°C) et de pH de la composition.

En ce qui concerne les motifs hydrophiles non ioniques qui peuvent être présents dans le polymère amphiphile, on peut citer notamment ceux obtenus à partir des monomères suivants :
- les amides des acides mono- ou poly- carboxyliques, linéaires, ramifiés, cycliques ou aromatiques, comprenant au moins une insaturation éthylénique ou dérivés ;
- les esters vinyliques permettant d'obtenir des blocs alcool polyvinylique après hydrolyse,
seuls ou en mélanges.

En ce qui concerne les monomères hydrophiles non ioniques, on peut citer tout particulièrement le (méth)acrylamide, le N-méthylol (méth)acrylamide, le (méth)acrytate de 2-hydroxyéthyle, les esters vinyliques permettant d'obtenir des blocs alcool polyvinylique après hydrolyse, comme l'acétate de vinyle, le Versatate^{®} de vinyle, le propionate de vinyle.

Pour ce qui a trait plus spécialement aux monomères à partir desquels on peut introduire des motifs hydrophiles cationiques dans le polymère amphiphile, on peut citer entre autres :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, la vinylamine, l'éthylène imine :

- les sels d'ammonium de diallyldialkyl ;
seuls ou en mélanges, ainsi que leurs sels, dans lesquels les groupes alkyles peuvent comprendre moins de 4 atomes de carbone, de préférence pas plus de 2.

S'ils se présentent sous la forme de sels, ces derniers sont de préférence choisis de telle sorte que le contre-ion soit un halogénure comme par exemple un chlorure, ou un sulfate, un hydrosulfate, un alkylsulfate (par exemple comprenant 1 à 6 atomes de carbone), un phosphate, un citrate, un formate, un acétate.

A titre d'exemples de monomères cationiques convenables figurent les monomères suivants :
- diméthyl amino éthyl (méth)acrylate, diméthyl amino propyl (méth)acrylate le ditertiobutyl aminoéthyl (méth)acrylate, le diméthyl amino méthyl (méth)acrylamide, le diméthyl amino propyl (méth)acrylamide ;
- l'éthylène imine, la vinylamine, la 2-vinylpyridine, la 4-vinylpyridine ;
- le chlorure de triméthylammonium éthyl (méth)acrylate, le méthyl sulfate de triméthylammonium éthyl acrylate, le chlorure de benzyl diméthylammonium éthyl (méth)acrylate, le chlorure de 4-benzoylbenzyl diméthyl ammonium éthyl acrylate, le chlorure de triméthyl ammonium éthyl (méth)acrylamido, le chlorure de triméthyl ammonium de vinylbenzyle ;
- le chlorure d'ammonium de diallyldiméthyl ;
seuls ou en mélanges, ou leurs sels correspondants.

En ce qui concerne les motifs hydrophiles anioniques, ceux-ci peuvent être obtenus à partir des monomères suivants, ainsi que leurs sels correspondants, leurs précurseurs, leurs dérivés phosphoniques ou sulfoniques :
- les acides mono- ou poly- carboxyliques linéaires, ramifiés, cycliques ou aromatiques, les dérivés N-substitués de tels acides ; les monoesters d'acides polycarboxyliques, comprenant au moins une insaturation éthylénique ;
- les acides vinyl carboxyliques linéaires, ramifiés, cycliques ou aromatiques ;
- les aminoacides comprenant une ou plusieurs insaturations éthyléniques ;
seuls ou en mélanges.

De manière avantageuse, on peut mettre en oeuvre les monomères suivants, sans intention de s'y limiter :
- l'acide acrylique, l'acide méthacrylique, l'acide fumarique, l'acide itaconique, l'acide citraconique, l'acide maléique, l'acide acrylamido glycolique, l'acide 2-propène 1-sulfonique, l'acide méthallyl sulfonique, l'acide styrène sulfonique, l'acide α-acrylamido méthylpropane sulfonique, le 2-sulfoéthylène méthacylate, l'acide sulfopropyl acrylique, l'acide bis-sulfopropyl acrylique, l'acide bis-sulfopropyl méthacrylique, l'acide sulfatoéthyl méthacrylique, le monoester phosphate d'acide hydroxyéthyl méthacrylique, ainsi que les sels de métal alcalin, comme le sodium, le potassium, ou d'ammonium ;
- l'acide vinyl sulfonique, l'acide vinylbenzène sulfonique, l'acide vinyl phosphonique, l'acide vinylidène phosphorique, l'acide vinyl benzoïque, ainsi que les sels de métal alcalin, comme le sodium, le potassium, ou d'ammonium ;
- le N-méthacryloyl alanine, le N-acryloyl-hydroxy-glycine ;
seuls ou en mélanges.

Pour ce qui a trait aux monomères amphotères, on peut mentionner à titre illustratif, les monomères du type des sulfobétaïnes, comme par notamment les (méth)acrylate d'alkyl dialkylammonium alkylsulfonate, du type des (méth)acrylate d'éthyl diméthylammonium propylsulfonate ; les (méth)acrylamide d'alkyl dialkylammonium sulfoalkyle, du type des (méth)acrylamide de propyl diméthylammonium sulfopropyle ; les vinyl pyridinium dialkylammonium sulfoalkyle, du type des vinyl pyridinium diméthylammonium sulfopropyle., seuls ou en mélange ou sous la forme de macromonomères.

Il est à noter, que l'on ne sortirait pas du cadre de la présente invention en mettant en oeuvre des monomères précurseurs de ceux qui viennent d'être cités. En d'autres termes, ces monomères présentent des motifs qui, une fois incorporés dans la chaîne polymère, peuvent être transformés, notamment par un traitement chimique tel que l'hydrolyse, pour redonner les espèces anioniques précitées. Par exemple, les monomères totalement ou partiellement estérifiés des monomères précités peuvent être mis en oeuvre pour être, par la suite, hydrolysés totalement ou en partie.

La masse molaire en nombre du polymère est habituellement supérieure ou égale à 1000 g/mol. Par ailleurs, la masse molaire en nombre est de préférence comprise entre 5000 et 100000 g/mol. Il est à noter que les masses molaires sont des masses absolues déterminées par chromatographie par exclusion stérique, couplée à une analyse MALLS (multi-angle laser light scattering).

Le polymère mis en oeuvre comme agent épaississant peut être obtenu de selon diverses méthodes de synthèse, les polymérisations classiques par voie radicalaire étant particulièrement avantageuses.

La polymérisation a de préférence lieu en phase aqueuse.

Elle consiste, de manière habituelle, à introduire les différents monomères dans une solution aqueuse.

Il peut de plus être approprié d'ajouter au mélange réactionnel un initiateur de polymérisation, même si cela ne représente pas une condition nécessaire. En effet, certains monomères, tels que le styrène, peuvent constituer en tant que tels une source de radicaux libres si la température de réaction est suffisamment élevée, généralement supérieure à 100°C.

Parmi les initiateurs de polymérisation radicalaire usuels, on peut mentionner par exemple les peroxydes d'hydrogène (l'hydroperoxyde de butyle tertiaire, l'hydroperoxyde de cumène, le t-butyl-peroxyacétate, le t-butyl-peroxybenzoate, le t-butylperoxyoctoate, le t-butylperoxynéodécanoate, le t-butylperoxyisobutarate, le peroxyde de lauroyle, le t-amylperoxypivalte, le t-butylperoxypivalate, le peroxyde de dicumyl, le peroxyde de benzoyle, le persulfate de potassium, le persulfate d'ammonium) ; les composés azoïques (le 2-2'-azobis(isobutyronitrile), le 2,2'-azobis(2-butanenitrile), le 4,4'-azobis(4-acide pentanoïque), le 1,1'-azobis(cyclohexane-carbonitrile), le 2-(t-butylazo)-2-cyanopropane, le 2,2'-azobis[2-méthyl-N-(1,1)-bis (hydroxyméthyl)-2-hydroxyéthyl] propionamide, le 2,2'-azobis(2-méthyl-N-hydroxyéthyl]-propionamide, le dichlorure de 2,2'-azobis(N,N'-diméthylèneisobutyramidine), le dichlorure de 2,2'-azobis (2-amidinopropane), le 2,2'-azobis (N,N'-diméthylène isobutyramide), le 2,2'-azobis(2-méthyl-N-[1,1-bis (hydroxyméthyl)-2-hydroxyéthyl] propionamide), le 2,2'-azobis(2-méthyl-N-[1,1-bis (hydroxyméthyl)éthyl] propionamide), le 2,2'-azobis[2-méthyl-N-(2-hydroxyéthyl) propionamide], le 2,2'-azobis(isobutyramide) dihydrate) ; - les systèmes redox comportant des combinaisons telles que les mélanges de peroxyde d'hydrogène, d'alkyle, peresters, percarbonates et similaires et de n'importe lequel des sels de fer, de sels titaneux, formaldéhyde sulfoxylate de zinc ou formaldéhyde sulfoxylate de sodium, et des sucres réducteurs, les persulfates, perborate ou perchlorate de métaux alcalins ou d'ammonium en association avec un bisulfite de métal alcalin, tel que le métabisulfite de sodium, et des sucres réducteurs, les persulfates de métal alcalin en association avec un acide arylphosphinique, tel que l'acide benzène phosphonique et autres similaires, et des sucres réducteurs.

La quantité d'initiateur à utiliser est déterminée de manière à ce que la quantité de radicaux produits soit d'au plus 50 % en mole, de préférence d'au plus 20 % en mole, par rapport à la quantité de monomères présents.

La température peut varier entre la température ambiante et 150°C selon la nature des monomères utilisés.

Comme indiqué auparavant, le polymère amphiphile est utilisé avec une teneur telle que la viscosité de la composition aqueuse comprenant le polymère amphiphile est au moins trois fois supérieure à celle de la composition dépourvue de polymère amphiphile et à celle d'une solution aqueuse comprenant le polymère amphiphile seul, dans les mêmes conditions de température, pH, force ionique et concentration (en d'autres termes, les concentrations des divers composés sont les mêmes que dans la composition). De manière avantageuse, la viscosité de la composition aqueuse comprenant le polymère amphiphile est au moins cinq fois supérieure, et de préférence au moins dix fois supérieure à celle de la composition dépourvue de polymère amphiphile et à celle d'une solution aqueuse comprenant le polymère amphiphile seul.

La viscosité est mesurée au moyen d'un viscosimètre de type Carrimed, avec une géométrie à cône-plan ; la mesure est effectuée à 25°C avec un gradient de cisaillement de 1 s⁻¹.

A titre illustratif, la quantité de polymère amphiphile entrant dans la composition aqueuse représente 0,05 à 5 % en poids de la composition aqueuse, de préférence entre 0,1 et 2 % en poids de la composition aqueuse.

Le composition aqueuse tensioactive comprenant le polymère amphiphile peut être employée dans de nombreux domaines d'applications, mais de préférence la composition aqueuse est une composition cosmétique, plus particulièrement destinée à être rincée.

Un autre objet de la présente est constitué par une composition aqueuse comprenant au moins 5 % en poids d'un ou plusieurs tensioactif(s) organisé(s) en phase comprenant des micelles géantes dont la longueur moyenne représente au moins 4 fois le diamètre moyen, et comprenant au moins un polymère amphiphile statistique choisi parmi ceux qui, au pH de la composition, sont solubles dans la composition aqueuse ; ledit polymère étant utilisé avec une teneur telle que la viscosité de la composition aqueuse comprenant le polymère est au moins trois fois supérieure à celle de la composition dépourvue de polymère et à elle d'une solution aqueuse comprenant le polymère seul. Plus particulièrement, la viscosité de la composition aqueuse comprenant le polymère amphiphile est au moins cinq fois supérieure, et de préférence au moins dix fois supérieure à celle de la composition dépourvue de polymère amphiphile et à celle d'une solution aqueuse comprenant le polymère amphiphile seul.

Plus particulièrement le polymère amphiphile présente un coefficient un coefficient f d'au moins 0,05, plus particulièrement compris entre 0,05 et 2, et un coefficient Δf compris entre -0,5 et 0,5.

Tout ce qui a été indiqué plus haut à propos du polymère amphiphile, notamment les motifs qui le constituent, leurs proportions, les voies de synthèse, reste valable et en sera pas repris à nouveau.

Selon un mode de réalisation de l'invention, la composition aqueuse comprend une teneur en polymère amphiphile comprise entre 0,05 à 5 % en poids de la composition aqueuse, de préférence entre 0,1 et 2 % en poids de la composition aqueuse. La teneur en tensioactif est quant à elle généralement comprise entre 5% et 20%, par exemple entre 5% et 10% ou entre 10% et 20%.

De plus la composition aqueuse est, de manière avantageuse, une composition cosmétique destinée à être rincée, telle qu'un shampoing. Dans de telles compositions la teneur en tensioactif est de préférence comprise entre 10 et 20%. Elle est typiquement de 12%, mais peut être ou supérieure ou inférieure à cette valeur.

Selon une autre possibilité, la composition aqueuse est utilisable dans le domaine de l'exploitation des gisements pétroliers ou de gaz. Il peut par exemple s'agit d'un fluide de forage ou d'un fluide de fracturation.

Des exemples concrets mais non limitatifs de l'invention vont maintenant être présentés.

### EXEMPLES

### 1/ Préparation du polymère statistique

### Structure et composition du polymère amphiphile P1 :

Le polymère est un copolymère statistique comprenant les motifs monomères suivants :
- Acrylate d'éthylhéxyle (EHA)
- Acide acrylique (AA)
- Sulfate de méthylacrylamidopropyl triméthylammonium (MES)

Le ratio molaire moyen de ces différents motifs dans le polymère est de :
24% d'EHA, 53% d'AA, 23% de MES

### Synthèse du polymère P1 :

La concentration théorique en polymère est de 20% dans un mélange éthanol:eau de 2:1.

La synthèse se fait selon le procédé batch suivant :

Dans un réacteur de 1 litre on ajoute :

| | |
|---|---|
| Eau : | 195,5 g |
| Ethanol : | 426,7 g |
| Acide acrylique: | 40,5 g |
| Sulfate de méthylacrylamidopropyl triméthyl | |
| ammonium (solution à 80%) : | 89,3 g |
| Acrylate d'éthylhéxyle : | 48,0 g |

A t₀ le mélange est chauffé à 75°C sous courant d'azote.

A t₀ + 1 on ajoute 0,32g d'initiateur d'azo bis-isobutyronitrile (AIBN ; Vazo 64) dans 5 g d'éthanol à 75°C.

A t₀+ 3 on ajoute de nouveau 0,16g d'initiateur AIBN (Vazo 64) dans 5 g d'éthanol. On augmente la température à 80°C.

A t₀ + 5 on commence à distiller progressivement l'éthanol à 83°C en ajoutant de l'eau

on distille à 95°C jusqu'à ce qu'il n'y ait plus de distillat.

La réaction est stoppée.

Le taux de monomères résiduel est contrôlé. Si ce taux est trop élevé on peut ajouter du persulfate de sodium jusqu'à l'obtention d'un taux de monomères résiduels acceptable.

Le produit est transparent. La concentration en polymère est de 40%. Le pH est de 2,18.

### 2/ Utilisation du polymère dans des formulations pour shampoing

### Composition des formulations shampoing :

### Formule A comparative (sans polymère) :

Il s'agit d'une formulation aqueuse de micelles géantes dans l'eau distillée, contenant un mélange suivant de tensioactifs et de sel monovalent :

| | |
|---|---|
| Lauryl éther sulfate de sodium | 14 % poids |
| Empicol ESB3M (Albright & Wilson) | |
| Cocamidopropyl bétaïne | 2 % poids |
| Tégobétaïne L7 (Goldschmidt) | |
| Chlorure de sodium | 1 % poids |
| (Fluka) | |

La formulation est obtenue par simple mélange à 25°C des constituants précédents.

On prépare ensuite quatre échantillons à des pH différents (2, 5, 7 et 10), ce dernier étant ajusté par ajout de soude concentrée (NaOH 1 M) ou d'acide chlorydrique concentré (HCl 1M).

Dans cette formule, les tensioactifs sont organisés en micelles géantes qui sont visualisées en microscopie électronique.

En outre, la formulation correspondante est visco-élastique, biréfringente sous écoulement (mesure faite dans un appareil Rheooptics).

### Formulation B selon l'invention (avec polymère) :

La formulation aqueuse dans l'eau distillée, comprend le mélange suivant de tensioactifs, de sel monovalent et de polymère P1 obtenu précédemment :

| | |
|---|---|
| Lauryl éther sulfate de sodium | 14 % poids |
| Empicol ESB3M (Albright & Wilson) | |
| Cocamidopropyl bétaïne | 2 % poids |
| Tégobétaïne L7 (Goldschmidt) | |
| Chlorure de sodium | 1 % poids |
| (Fluka) | |
| Polymère P1 | 1 % poids |

On prépare quatre échantillons, comprenant les tensioactifs et le sel, à des pH différents (2, 5, 7 et 10) en procédant comme pour la formulation A.

Le polymère P1 est mis en solution aqueuse séparément au même pH que la formulation dans laquelle il doit être ajouté.

La formulation est obtenue par mélange de cette solution de polymère au premier mélange.

### Performance rhéologique des deux formulations

Les mesures de viscosités sont effectuées sur CARRIMED à une température de 25°C sur les formulations de shampoings A et B préparés à différents pH.

On reporte dans le tableau suivant les viscosités (en mPa.s) mesurées pour un gradient de cisaillement fixe, y =10 s⁻¹.

| pH | 2 | 5 | 7 | 10 |
|---|---|---|---|---|
| Viscosité du Shampoing A | 150 | 43 | 27 | 18 |
| Viscosité du Shampoing B | 12000 | 4750 | 5000 | 1250 |

L'ajout de polymère entraîne un gain en viscosité et ce, quel que soit le pH considéré dans cet exemple.

## Revendications

1. Utilisation comme agent épaississant d'une composition aqueuse comprenant au moins 5 % en poids d'un ou plusieurs tensioactif(s) organisé(s) en phase comprenant des micelles géantes dont la longueur moyenne représente au moins 4 fois le diamètre moyen, d'au moins un polymère amphiphile statistique chargé soluble dans la composition aqueuse ; ledit polymère amphiphile étant utilisé avec une teneur telle que la viscosité de la composition aqueuse comprenant le polymère amphiphile est au moins trois fois supérieure à celle de la composition dépourvue de polymère amphiphile et à celle d'une solution aqueuse comprenant le polymère amphiphile seul.

2. Utilisation selon la revendication précédente, **caractérisé en ce que** le polymère amphiphile présente un coefficient f d'au moins 0,05.

3. Utilisation selon l'une des revendications précédentes, **caractérisée en ce que** le polymère amphiphile comprend une charge nette neutre.

4. Utilisation selon l'une des revendications 1 ou 2, **caractérisée en ce que** le polymère amphiphile comprend une charge nette opposée à celle du ou des tensioactifs organisés en phase comprenant des micelles géantes.

5. Utilisation selon l'une des revendications précédentes, **caractérisée en ce que** le polymère amphiphile présente une valeur de Δf comprise entre -0,5 et 0,5.

6. Utilisation selon l'une des revendications précédentes, **caractérisée en ce que** le polymère comprend des motifs hydrophobes comprenant au moins 6 atomes de carbone, de type alkyle, linéaire ou ramifié, ou aryle, éventuellement porteur d'un ou plusieurs radicaux alkyles, linéaires ou non.

7. Utilisation selon la revendication précédente, **caractérisée en ce que** le nombre de moles théorique de monomères hydrophobes présent dans la composition du polymère amphiphile est d'au moins 5 % par rapport au nombre de moles total théorique de monomères du polymère.

8. Utilisation selon l'une des revendications 6 ou 7, **caractérisée en ce que** les motifs hydrophobes sont des radicaux alkyles, et **en ce que** le nombre de moles théorique de monomères hydrophobes présent dans la composition du polymère amphiphile est compris entre 10 et 50% en poids par rapport au nombre de moles total théorique de monomères du polymère, de préférence entre 25 et 40 %.

9. Utilisation selon l'une des revendications 6 ou 7, **caractérisée en ce que** les motifs hydrophobes sont des radicaux aryles, le nombre de moles théorique de monomères hydrophobes présent dans la composition du polymère amphiphile est compris entre 5 et 15% en poids par rapport au nombre de moles total théorique de monomères du polymère.

10. Utilisation selon l'une des revendications précédentes, **caractérisée en ce que** le polymère amphiphile comprend des motifs hydrophiles non ioniques obtenus à partir des monomères suivants :
- les amides des acides mono- ou poly- carboxyliques, linéaires, ramifiés, cycliques ou aromatiques, comprenant au moins une insaturation éthylénique ou dérivés ;
- les esters vinyliques permettant d'obtenir des blocs alcool polyvinylique après hydrolyse,
seuls ou en mélanges.

11. Utilisation selon l'une des revendications précédentes, **caractérisée en ce que** le polymère amphiphile comprend des motifs hydrophiles cationiques obtenus à partir des monomères suivants, ainsi que leurs sels correspondants :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, la vinylamine, l'éthylène imine ;
- les sels d'ammonium de diallyldialkyl ;
seuls ou en mélanges.

12. Utilisation selon l'une des revendications précédentes, **caractérisée en ce que** le polymère amphiphile comprend des motifs hydrophiles anioniques obtenus à partir des monomères suivants, ainsi que leurs sels correspondants, leurs précurseurs, leurs dérivés phosphoniques ou sulfoniques :
- les acides mono- ou poly- carboxyliques linéaires, ramifiés, cycliques ou aromatiques, les dérivés N-substitués de tels acides ; les monoesters d'acides polycarboxyliques, comprenant au moins une insaturation éthylénique ;
- les acides vinyl carboxyliques linéaires, ramifiés, cycliques ou aromatiques ;
- les aminoacides comprenant une ou plusieurs insaturations éthyléniques ;
seuls ou en mélanges.

13. Utilisation selon l'une des revendications précédentes, **caractérisée en ce que** la teneur en polymère amphiphile représente 0,05 à 5 % en poids de la composition aqueuse, de préférence entre 0,1 et 2 % en poids de la composition aqueuse.

14. Utilisation selon l'une des revendications précédentes, **caractérisée en ce que** la composition aqueuse est une composition cosmétique, plus particulièrement destinée à être rincée.

15. Utilisation selon l'une des revendications précédentes, **caractérisée en ce que** la composition aqueuse est une composition utilisable dans l'exploitation de gisements pétroliers ou de gaz..

16. Composition aqueuse comprenant au moins 5 % en poids d'un ou plusieurs tensioactif(s) organisé(s) en phase comprenant des micelles géantes dont la longueur moyenne représente au moins 4 fois le diamètre moyen, et comprenant au moins un polymère amphiphile statistique choisi parmi ceux qui, au pH de la composition, sont solubles dans la composition aqueuse ; ledit polymère étant utilisé avec une teneur telle que la viscosité de la composition aqueuse comprenant le polymère est au moins trois fois supérieure à celle de la composition dépourvue de polymère et à elle d'une solution aqueuse comprenant le polymère seul.

17. Composition selon la revendication précédente, **caractérisé en ce que** le polymère amphiphile présente
- une valeur du paramètre f, correspondant à la somme du nombre de moles théorique de monomères portant une charge dans le polymère, divisée par la somme du nombre de moles total théorique de monomères dans le polymère, d'au moins 0,05, et
- une valeur de Δf, correspondant à la différence entre le nombre de moles théoriques de monomères cationique et le nombre de moles théoriques de monomères anioniques du polymère, divisée par la somme du nombre de moles théorique total du polymère, est comprise entre -0,5 et 0,5.

18. Composition selon l'une des revendications 16 ou 17, **caractérisée en ce que** le polymère amphiphile comprend une charge nette neutre ou de charge opposée à celle du ou des tensioactifs organisés en phase comprenant des micelles géantes.

19. Composition selon l'une des revendications 16 à 18, **caractérisée en ce que** le nombre de moles théorique de monomères hydrophobes présent dans la polymère est d'au moins 5 % par rapport au nombre de mole théorique total de monomères présents dans le polymère.

20. Composition selon l'une des revendications 16 à 19, **caractérisée en ce que** la composition aqueuse est une composition cosmétique destinée à être rincée,

21. Composition selon l'une des revendications 16 à 19, **caractérisée en ce que** la composition aqueuse est une composition utilisable dans l'exploitation de gisements pétroliers ou de gaz.

22. Composition selon l'une des revendications 16 à 21, **caractérisée en ce que** la teneur en polymère amphiphile représente 0,05 à 5 % en poids de la composition aqueuse, de préférence entre 0,1 et 2 % en poids de la composition aqueuse.

## Claims

1. The use, as thickening agent for an aqueous composition comprising at least 5% by weight of one or more surfactant(s) organized as a phase comprising giant micelles, the mean length of which represents at least 4 times the mean diameter, of at least one charged random amphiphilic polymer which is soluble in the aqueous composition; said amphiphilic polymer being used with a content such that the viscosity of the aqueous composition comprising the amphiphilic polymer is at least three times greater than that of the composition devoid of amphiphilic polymer and than that of an aqueous solution comprising the amphiphilic polymer alone.

2. The use as claimed in the preceding claim, **characterized in that** the amphiphilic polymer exhibits a coefficient f of at least 0.05.

3. The use as claimed in either of the preceding claims, **characterized in that** the amphiphilic polymer comprises a neutral net charge.

4. The use as claimed in either of claims 1 and 2, **characterized in that** the amphiphilic polymer comprises a net charge opposite that of the surfactant or surfactants organized as a phase comprising giant micelles.

5. The use as claimed in one of the preceding claims, **characterized in that** the amphiphilic polymer exhibits a value of Δf of between -0.5 and 0.5.

6. The use as claimed in one of the preceding claims, **characterized in that** the polymer comprises hydrophobic units comprising at least 6 carbon atoms of the following type: linear or branched alkyl or aryl optionally carrying one or more linear or nonlinear alkyl radicals.

7. The use as claimed in the preceding claim, **characterized in that** the theoretical number of moles of hydrophobic monomers present in the composition of the amphiphilic polymer is at least 5% with respect to the total theoretical number of moles of monomers of the polymer.

8. The use as claimed in either of claims 6 and 7, **characterized in that** the hydrophobic units are alkyl radicals and **in that** the theoretical number of moles of hydrophobic monomers present in the composition of the amphiphilic polymer is between 10 and 50%, preferably between 25 and 40%, by weight with respect to the total theoretical number of moles of monomers of the polymer.

9. The use as claimed in either of claims 6 and 7, **characterized in that** the hydrophobic units are aryl radicals, the theoretical number of moles of hydrophobic monomers present in the composition of the amphiphilic polymer is between 5 and 15% by weight with respect to the total theoretical number of moles of monomers of the polymer.

10. The use as claimed in one of the preceding claims, **characterized in that** the amphiphilic polymer comprises nonionic hydrophilic units obtained from the following monomers:
- amides of linear, branched, cyclic or aromatic mono- or polycarboxylic acids comprising at least one ethylenic unsaturation, or derivatives;
- vinyl esters which make it possible to obtain poly(vinyl) alcohol blocks after hydrolysis,
alone or as mixtures.

11. The use as claimed in one of the preceding claims, **characterized in that** the amphiphilic polymer comprises cationic hydrophilic units obtained from the following monomers, and their corresponding salts:
- aminoalkyl (meth)acrylates or aminoalkyl(meth)-acrylamides;
- monomers comprising at least one secondary, tertiary or quaternary amine functional group or a heterocyclic group comprising a nitrogen atom, vinylamine or ethylenimine;
- diallyldialkylammonium salts;
alone or as mixtures.

12. The use as claimed in one of the preceding claims, **characterized in that** the amphiphilic polymer comprises anionic hydrophilic units obtained from the following monomers, and their corresponding salts, their precursors or their phosphonic or sulfonic derivatives:
- linear, branched, cyclic or aromatic mono- or polycarboxylic acids or the N-substituted derivatives of such acids; monoesters of polycarboxylic acids, comprising at least one ethylenic unsaturation;
- linear, branched, cyclic or aromatic vinylcarboxylic acids;
- amino acids comprising one or more ethylenic unsaturations;
alone or a mixtures.

13. The use as claimed in one of the preceding claims, **characterized in that** the content of amphiphilic polymer represents 0.05 to 5% by weight of the aqueous composition, preferably between 0.1 and 2% by weight of the aqueous composition.

14. The use as claimed in one of the preceding claims, **characterized in that** the aqueous composition is a cosmetic composition more particularly intended to be rinsed out.

15. The use as claimed in one of the preceding claims, **characterized in that** the aqueous composition is a composition which can be used in the exploitation of oil or gas deposits.

16. An aqueous composition comprising at least 5% by weight of one or more surfactant(s) organized as a phase comprising giant micelles, the mean length of which represents at least 4 times the mean diameter, and comprising at least one random amphiphilic polymer chosen from those which, at the pH of the composition, are soluble in the aqueous composition; said polymer being used with a content such that the viscosity of the aqueous composition comprising the polymer is at least three times greater than that of the composition devoid of polymer and than that of an aqueous solution comprising the polymer alone.

17. The composition as claimed in the preceding claim, **characterized in that** the amphiphilic polymer exhibits
- a value of the parameter f, corresponding to the sum of the theoretical number of moles of monomers carrying a charge in the polymer, divided by the sum of the total theoretical number of moles of monomers in the polymer, of at least 0.05, and
- a value of Δf, corresponding to the difference between the theoretical number of moles of cationic monomers and the theoretical number of moles of anionic monomers of the polymer, divided by the sum of the total theoretical number of moles of the polymer, is between -0.5 and 0.5.

18. The composition as claimed in either of claims 16 and 17, **characterized in that** the amphiphilic polymer comprises a neutral net charge or a charge opposite that of the surfactant or surfactants organized as a phase comprising giant micelles.

19. The composition as claimed in one of claims 16 to 18, **characterized in that** the theoretical number of moles of hydrophobic monomers present in the polymer is at least 5% with respect to the total theoretical number of moles of monomers present in the polymer.

20. The composition as claimed in one of claims 16 to 19, **characterized in that** the aqueous composition is a cosmetic composition intended to be rinsed out.

21. The composition as claimed in one of claims 16 to 19, **characterized in that** the aqueous composition is a composition which can be used in the exploitation of oil or gas deposits.

22. The composition as claimed in one of claims 16 to 21, **characterized in that** the content of amphiphilic polymer represents 0.05 to 5% by weight of the aqueous composition, preferably between 0.1 and 2% by weight of the aqueous composition.

## Patentansprüche

1. Verwendung als Verdickungsmittel einer wässrigen Zusammensetzung, umfassend wenigstens 5 Gew.-% eines Tensids oder mehrerer Tenside, die als Phase organisiert sind, die Riesenmizellen umfasst, deren mittlere Länge wenigstens das Vierfache des mittleren Durchmessers beträgt, wenigstens ein geladenes statistisches amphiphiles Polymer, das in der wässrigen Zusammensetzung löslich ist; wobei das amphiphile Polymer mit einem derartigen Gehalt verwendet wird, dass die Viskosität der wässrigen Zusammensetzung, die das amphiphile Polymer umfasst, wenigstens dreimal höher ist als jene der Zusammensetzung ohne das amphiphile Polymer und als jene einer wässrigen Zusammensetzung, die nur das amphiphile Polymer umfasst.

2. Verwendung gemäß dem vorausgegangenen Anspruch, **dadurch gekennzeichnet, dass** das amphiphile Polymer einen Koeffizienten f von wenigstens 0,05 aufweist.

3. Verwendung gemäß einem der vorausgegangenen Ansprüche, **dadurch gekennzeichnet, dass** das amphiphile Polymer eine neutrale Nettoladung aufweist.

4. Verwendung gemäß einem der Ansprüche 1 oder 2, **dadurch gekennzeichnet, dass** das amphiphile Polymer eine Nettoladung aufweist, die entgegengesetzt zu jener des Tensids oder der Tenside ist, die als Phase mit Riesenmizellen organisiert sind.

5. Verwendung gemäß einem der vorausgegangenen Ansprüche, **dadurch gekennzeichnet, dass** das amphiphile Polymer einen Wert Δf zwischen -0,5 und 0,5 aufweist.

6. Verwendung gemäß einem der vorausgegangenen Ansprüche, **dadurch gekennzeichnet, dass** das Polymer hydrophobe Einheiten mit wenigstens 6 Kohlenstoffatomen aufweist, der Art Alkyl, linear oder verzweigt, oder Aryl, gegebenenfalls Träger eines oder mehrerer Alkylradikale, linear oder nicht.

7. Verwendung gemäß dem vorausgegangenen Anspruch, **dadurch gekennzeichnet, dass** die theoretische Molzahl an hydrophoben Monomeren, die in der Zusammensetzung des amphiphilen Polymers vorhanden ist, wenigstens 5% bezogen auf die theoretische Gesamtmolzahl an Monomeren des Polymers beträgt.

8. Verwendung gemäß einem der Ansprüche 6 oder 7, **dadurch gekennzeichnet, dass** die hydrophoben Einheiten Alkylradikale sind, und **dadurch**, dass die theoretische Molzahl an hydrophoben Monomeren, die in der Zusammensetzung des amphiphilen Polymers vorhanden sind, zwischen 10 und 50 Gew.-% bezogen auf die theoretische Gesamtmolzahl an Monomeren des Polymers, vorzugsweise zwischen 25 und 40%, beträgt.

9. Verwendung gemäß einem der Ansprüche 6 oder 7, **dadurch gekennzeichnet, dass** die hydrophoben Einheiten Arylradikale sind, die theoretische Molzahl an hydrophoben Monomeren, die in der Zusammensetzung des amphiphilen Polymers vorhanden sind, zwischen 5 und 15 Gew.-% bezogen auf die theoretische Gesamtmolzahl an Monomeren des Polymers beträgt.

10. Verwendung gemäß einem der vorausgegangenen Ansprüche, **dadurch gekennzeichnet, dass** das amphiphile Polymer nicht ionische hydrophile Einheiten umfasst, die aus den folgenden Monomeren erhalten werden:
- Amiden von Mono- oder Polycarbonsäuren, linear, verzweigt, zyklisch oder aromatisch, die wenigstens einfach ethylenisch ungesättigt sind, oder deren Derivaten;
- Vinylestern, die es ermöglichen, nach Hydrolyse Polyvinylalkoholblöcke zu erhalten,
einzeln oder als Gemische.

11. Verwendung gemäß einem der vorausgegangenen Ansprüche, **dadurch gekennzeichnet, dass** das amphiphile Polymer kationische hydrophile Einheiten umfasst, die aus den folgenden Monomeren sowie ihren entsprechenden Salzen erhalten werden:
- Aminoalkyl(meth)acrylaten, Aminoalkyl(meth)acrylamiden;
- Monomeren mit wenigstens einer sekundären, tertiären oder quaternären Aminfunktion, oder einer heterozyklischen Gruppe, die ein Stickstoffatom enthält, Vinylamin, Ethylenimin;
- Diallyldialkylammoniumsalzen;
einzeln oder als Gemische.

12. Verwendung gemäß einem der vorausgegangenen Ansprüche, **dadurch gekennzeichnet, dass** das amphiphile Polymer anionische hydrophile Einheiten umfasst, die aus den folgenden Monomeren sowie ihren entsprechenden Salzen, ihren Vorläufern, ihren Phosphon- oder Sulfonderivaten erhalten werden:
- Mono- oder Polycarbonsäuren, linear, verzweigt, zyklisch oder aromatisch, den N-substituierten Derivaten dieser Säuren, Monoestern von Polycarbonsäuren, die wenigstens einfach ethylenisch ungesättigt sind;
- Vinylcarbonsäuren, linear, verzweigt, zyklisch oder aromatisch;
- Aminosäuren, die einfach oder mehrfach ethylenisch ungesättigt sind;
einzeln oder als Gemische.

13. Verwendung gemäß einem der vorausgegangenen Ansprüche, **dadurch gekennzeichnet, dass** der Gehalt an amphiphilem Polymer 0,05 bis 5 Gew.-% der wässrigen Zusammensetzung, vorzugsweise zwischen 0,1 und 2 Gew.-% der wässrigen Zusammensetzung darstellt.

14. Verwendung gemäß einem der vorausgegangenen Ansprüche, **dadurch gekennzeichnet, dass** die wässrige Zusammensetzung eine kosmetische Zusammensetzung ist, genauer dazu bestimmt, abgespült zu werden.

15. Verwendung gemäß einem der vorausgegangenen Ansprüche, **dadurch gekennzeichnet, dass** die wässrige Zusammensetzung eine Zusammensetzung ist, die bei der Ausbeutung von Erdöl- oder Gasquellen verwendbar ist.

16. Wässrige Zusammensetzung mit wenigstens 5 Gew.-% eines Tensids oder mehrerer Tenside, die als Phase organisiert sind, die Riesenmizellen umfasst, deren mittlere Länge wenigstens das Vierfache des mittleren Durchmessers beträgt, und mit wenigstens einem statistischen amphiphilen Polymer, das aus jenen gewählt ist, die bei dem pH der Zusammensetzung in der wässrigen Zusammensetzung löslich sind; wobei das amphiphile Polymer mit einem derartigen Gehalt verwendet wird, dass die Viskosität der wässrigen Zusammensetzung, die das Polymer umfasst, wenigstens dreimal höher ist als jene der Zusammensetzung ohne das Polymer und als jene einer wässrigen Zusammensetzung, die nur das Polymer umfasst.

17. Zusammensetzung gemäß dem vorausgegangenen Anspruch, **dadurch gekennzeichnet, dass** das amphiphile Polymer aufweist
- einen Wert des Parameters f, der der Summe der theoretischen Molzahl an Monomeren, die in dem Polymer eine Ladung tragen, geteilt durch die Summe der theoretischen Gesamtmolzahl an Monomeren in dem Polymer entspricht, von wenigstens 0,05, und
- einen Wert Δf, der dem Unterschied zwischen der theoretischen Molzahl an kationischen Monomeren und der theoretischen Molzahl an anionischen Monomeren des Polymers, geteilt durch die Summe der theoretischen Gesamtmolzahl des Polymers entspricht, und zwischen -0,5 und 0,5 liegt.

18. Zusammensetzung gemäß einem der Ansprüche 16 oder 17, **dadurch gekennzeichnet, dass** das amphiphile Polymer eine neutrale Nettoladung oder eine Nettoladung auf weist, die entgegengesetzt zu jener des Tensids oder der Tenside ist, die als Phase mit Riesenmizellen organisiert sind.

19. Zusammensetzung gemäß einem der Ansprüche 16 bis 18, **dadurch gekennzeichnet, dass** die theoretische Molzahl an hydrophoben Monomeren, die in dem Polymer vorhanden ist, wenigstens 5% bezogen auf die theoretische Gesamtmolzahl an in dem Polymer vorhandenen Monomeren beträgt.

20. Zusammensetzung gemäß einem der Ansprüche 16 bis 19, **dadurch gekennzeichnet, dass** die wässrige Zusammensetzung eine kosmetische Zusammensetzung ist, dazu bestimmt, abgespült zu werden.

21. Zusammensetzung gemäß einem der Ansprüche 16 bis 19, **dadurch gekennzeichnet, dass** die wässrige Zusammensetzung eine Zusammensetzung ist, die bei der Ausbeutung von Erdöl- oder Gasquellen verwendbar ist.

22. Zusammensetzung gemäß einem der Ansprüche 16 bis 21, **dadurch gekennzeichnet, dass** der Gehalt an amphiphilem Polymer 0,05 bis 5 Gew.-% der wässrigen Zusammensetzung, vorzugsweise zwischen 0,1 und 2 Gew.-% der wässrigen Zusammensetzung darstellt.
